# EUROPEAN PATENT APPLICATION

(11) **EP 3 895 731 A1**
(43) Date of publication of application: **20.10.2021**
(21) Application number: 20000163.4
(22) Date of filing: 21.04.2020
(51) Int. Cl.: A61K 39/39, A61K 31/4745, A61K 31/739, A61P 31/12, A61P 37/04

(54) **METHOD FOR EMERGENCY IMMUNIZATION OF SELECTED PARTS OF A POPULATION**

(30) Priority: 19.04.2020 EP 20000159
(71) Applicant: Englmeier, Ludwig, 83080 Oberaudorf (DE)
(72) Inventor: Englmeier, Ludwig, 83080 Oberaudorf (DE)

(57) **Abstract**

The present invention relates to a tlr agonist for use in the one-time prophylactic treatment of a subpopulation against an infection, wherein the subpopulation is a collection of asymptomatic subjects in proximity to subjects of the population who show symptoms of infection.

The invention also relates to a method of providing immunity against a virus to a selected subpopulation of a larger population by administering an agonist of a toll-like-receptor (tlr) to individuals from the selected subpopulation in a first step, and then exposing the individuals who had been pretreated with the tlr agonist to the virus.

## Description

### FIELD OF THE INVENTION

The present invention relates to a method of providing immunity against a virus to selected parts of a population in an emergency situation

### BACKGROUND OF THE INVENTION

In December 2019, health authorities in Wuhan, China, identified a cluster of pneumonia cases of unknown aetiology linked to the city's South China Seafood Market. Subsequent investigations revealed a novel coronavirus, SARS-CoV-2, as the causative agent now at the heart of a major outbreak.

The clinical disease termed COVID-19 is caused by a novel betacoronavirus, now named SARS-CoV-2. SARS-CoV-2 shares 79% sequence identity with SARS-CoV, the virus which caused a major outbreak in 2002-2003. In common with SARS-CoV, SARS-CoV-2 utilises the ACE-2 receptor for cell entry. The most common symptoms being reported are fever, cough or chest tightness, and dyspnoea. Most cases are reported to experience a mild illness course (Lake, MA; Clin. Med. (Lond) 2020 Mar; 20(2):124-127), however, as of the priority date of this patent application, already about 150000 people have died from COVID-19 (https://www.worldometers.info/coronavirus/ on 17.4.2020).

In the absence of immunity against the new virus, infection with SARS-CoV-2 can spread exponentially in a population. This spread has been checked and contained in several countries, but harsh measures such as a "lockdown" or quarantaine-like mobility restrictions to people have been necessary to achieve this result.

But parts of the general population cannot stay at home because their work is essential for even a basic functioning of society. In particular medical personnel cannot just "stay at home", but is even exposed to a high risk of contracting the virus by caring for COVID-19 patients. Many doctors and nurses get infected with the virus despite the implementation of vigorous hygienic measures to protect against virus exposure.

It has been common sense that overcoming this disease would require the successful development of a vaccine against SARS-CoV-2. However, vaccine development against the closely related SARS-CoV-1 virus and against MERS has been so far unsuccessful, despite years of research. There is thus an urgent need for providing immunity against SARS-CoV-2 or future pandemic viruses.

It is of course most desirable to achieve immunization of a collection of individuals by a vaccination with minimal side effects which has undergone vigorous testing and clinical trials. Alas, this is often not available. In such a situation immunization of a collection of individuals - a subpopulation - among a larger population would only achievable if the subpopulation contracted the disease, with all dire consequences that this might have.

In the context of the current COVID-19 pandemic it has indeed been suggested that subpopulations having an infection fatality rate that is known to be or expected to be below the average infection fatality rate of the population, such as young adults up to the age of 40 years, should be allowed to contract the virus. And politicians might even decide that this will be done in the near future.

Of course, even though the fatality rate is low in the above-mentioned patient group, it is not zero, so some of the exposed young people would die from the exposure and a substantial fraction would come down with serious disease symptoms.

### SUMMARY OF THE INVENTION

The present invention relates to a method of providing immunity against a virus to a selected subpopulation of a larger population by administering an agonist of a toll-like-receptor (tlr) to individuals from the selected subpopulation in a first step, and then exposing the individuals who had been pretreated with the tlr agonist to the virus.

In the present Codiv-19 pandemia several groups apparently have a high risk of developing severe COVID-19: obese people, people of old age, men and patients suffering from systemic lupus erythematosus. The present inventor has identified a common denominator for these groups: low tlr7 activity either by low gene dosage, as in men (tlr7 escapes X-chromosome inactivation in women, who thus have a higher expression of tlr7 than men, or low tlr7 activity due to toll-like receptor tolerance in obese, old and SLE patients. Obese patients show a chronic activation of tlr7 (Revelo et al., Cell Rep. 2016 Jul 19;16(3):717-30) which leads to the development of tolerance to tlr7 signalling over time (Michaelis et al., Brain Behav Immun. 2019 Nov;82:338-353). People of old age overexpress miR-146a, leading to a constant stimulation of tlrs and consequently also to the development of tlr tolerance. SLE patients suffer from an excess of tlr7 substrates produced by by the body. Thus, it can be expected that also in this case the body will try to downregulate the sensitivity of toll-like receptor signalling. When a viral infection occurs in a situation of developed tlr tolerance, the initial response of the body to the infection will be delayed or insufficient - due to the downregulated sensitivity of tlrs - thus exposing the affected individuals to severe disease. These observations point towards tlr agonists as being of potential help in the Covid-19 pandemic, as will be explained below.

Without wishing to be bound to any theory, the pretreatment of the subpopulation with the tlr agonist will boost the innate immune system of the pretreated individuals, which in turn will help them to better cope with the virus as long as the immune-boost by the tlr agonist is still active. Exposure to the virus will then lead to an infection, which is, however, less severe, but still leads to an immune response in the individuals of the subpopulation and to subsequent immunization of the subpopulation.

Zhao et al., J. Virol. 2012 Nov, 86(21):11416-11424 have shown that intranasal treatment with poly(I:C) protects aged mice from lethal respiratory virus infections. They suggest poly (I:C) for further investigation for prophylaxis in aged populations at high risk. However, they do not suggest, after treatment of subjects with poly (I:C), to actually expose pretreated subjects to the virus for the purpose of creating immunity in the subjects. However, the results from Zhao et al. make it plausible that a pretreatment of a subpopulation with a tlr agonist, and in particular pretreatment of a subpopulation that is less vulnerable to the effects of the virus infection, would convey some protection to the pretreated subpopulation upon exposure to the virus in the period after the pretreatment when the immune-system is still boosted.

In the context of the present SARS-Cov-2 pandemic, the present invention will lower the already very low infection fatality rate and the low rate of severe COVID-19 in young adults below the age of 40 even further. While it will remain a difficult political decision, whether the remaining lowered fatality rate and rate of severe disease are acceptable from an ethical standpoint, the present invention at least provides a less undesirable option for creating immunity in a subpopulation than virus infection without pretreatment, for example in so-called "Corona parties".

The present invention also relates to the use of a toll-like receptor agonist in the immunization of a subpopulation of a population during a virus outbreak.

The present invention also relates to a one time administration of a tlr agonist for the immediate prophylactic treatment of a virus infection in a subpopulation, wherein the subpopulation is a collection of asymptomatic subjects in proximity to subjects of the population who show symptoms of virus infection.

### DETAILED DESCRIPTION OF THE INVENTION

The present invention relates to a method of providing immunity against a virus to a selected subpopulation of a larger population by
a) administering an agonist of a toll-like-receptor to individuals from the selected subpopulation, and then
b) exposing the individuals from step a) to the virus.

The administration of the toll-like receptor agonist shortly prior to the exposure to the virus activates the innate immune system of the subjects of the subpopulation and enables them to better deal with the virus once exposed to it in the period when the immune system is still boosted by the effect of the tlr agonist. The time between administration of the tlr agonist and exposure to the virus can vary for the tlr agonist to be used and is preferably at most seven days, more preferably at most 5 days and most preferably exposure to the virus is effected in the period from immediately after administration of the tlr agonist to three days after administration of the tlr agonist, such as from one hour to 3 days after administration of the tlr agonist.

"Population" means a group of individual organisms of the same species that live in the same geographic area. For example all human beings living in the United States of America form the "population" of the USA. Or all cows of a farm form the population of cows on the farm. "Subpopulation" means a proper subset of the population, wherein the individuals from the subset share at least one characteristic with one another that is not shared by all individuals of the population. For example, old people above the age of 60 in the USA would form a subpopulation within the larger population of the USA.

While the method of the present invention would be predicted to work for all organisms that have toll-like-receptors as part of their immune system and that have both an innate and an adaptive immune system, preferred populations are vertebrate populations and in particular mammalian populations. Preferred populations are human populations, and populations of farm animals, such as cow, pig, horse, goat or sheep.

Typically a subpopulation is chosen for immunization that has an infection fatality rate that is known to be or expected to be below the average infection fatality rate of the population. In the current Covid-19 epidemic, for example, it is known that children from age 2 to young adults to age 30 apparently have a lower risk to die from SARS-CoV-2 infection than old people above the age of 60. It has therefore been discussed to infect this group on purpose so as to generate a higher rate of immune people in the population and to eventually protect the older people via "herd immunity". However, it is felt that the mortality and in particular the rate of severe disease would still be too high for such an approach. The method of the present invention would make such an approach more viable as it would further decrease the already low mortality rate.

Another interesting subpopulation for the method of the present invention is a subpopulation that is at an increased risk of exposure to the virus, such as medical personnel. The numbers of infected doctors and nurses, who care for Covid-19-patients, are too high. In particular in situations where protective personal equipment is on short supply and where an increased rate of infection among medical personnel is to be, unfortunately, expected, it might be desirable to immunize this subpopulation before expected virus exposure.

While the method of the present invention is predicted to work against infections by all pathogens that can be recognized by the innate immune system, preferred pathogens to which immunity is to be conferred by the method of the present invention are viruses, and in particular - if only under the impression of the present pandemic - it is a virus where the virus causes a respiratory disease. The virus can be an RNA-virus, such as a coronavirus, and in particular a SARS-like virus like SARS-CoV-2.

Of course, if the population to be immunized changes, for example if a population of swine is to be immunized, also the virus changes correspondingly, for example for swine it might become desirable to immunize against against classical swine fever, caused by a pestivirus, or against African swine fever, caused by an asfarvirus.

While the method of the present invention is predicted to work also if the tlr agonist is administered systemically and later exposure to the virus is tissue-specific, it is preferred that exposure to the virus in step b) and administration of the toll-like-receptor agonist in step a) are effected to the same part of the body. For example, the part of the body can be the upper respiratory tract, such as the nasal cavity, which is a particularly desired combination of tlr agonist administration and virus exposure for respiratory viruses. For example, to be seen in the context of the current Covid-19 pandemic, the tlr agonist would first be administered to the nasal cavity (for example GSK2245035 is a tlr7 agonist in clinical trials for intranasal administration) and then shortly thereafter the SARS-CoV-2 virus would be administered to the nasal cavity as well.

Most toll-like receptors signal via MyD88 (or a mammalian homologue thereof) and activate the gene expression of antiviral cytokines and chemokines. For the method of the present invention, the nature of the tlr agonist can be selected so that it activates those toll like receptors that are involved in the individual's innate immune response against the pathogen against which immunization is to be effected. For example, when the population is a human population and the pathogen is a virus, the toll-like receptors tlr3, tlr7, tlr8 and tlr9 are known to be involved in the recognition of viral antigens.

The present invention can relate to a method, where the toll-like receptor agonist in step a) is selected to activate a toll-like receptor that is not involved in the innate immune response against the pathogen of step b).

But the present invention also relates to a method, where the toll-like receptor agonist in step a) is selected to activate a toll-like receptor that is involved in the innate immune response against the pathogen of step b). In the preferred situation of providing immunization against a virus, preferred tlr agonists are a tlr3 agonist, a tlr4 agonist, a tlr7 agonist, a tlr8 agonist and/or a tlr9 agonist. The invention also relates to a method wherein the tlr agonist is selected from the following groups: tlr3, tlr4 and tlr9; tlr3, tlr4, tlr7 and tlr8; tlr4, tlr7, tlr8 and tlr9; tlr7, tlr8 and tlr9; tlr3, tlr7 and tlr8; tlr4, tlr7 and tlr9.

Many tlr agonists have been described in the literature and formulations for systemic administration but also local administration have been described and sometimes also been developed to the stage of being tested in clinical trials.

A preferred tlr agonist is Poly (I:C). Polyinosinic:polycytidylic acid is a mismatched double-stranded RNA with one strand being a polymer of inosinic acid, the other strand a polymer of cytidilic acid. It is a strong agonist of tlr3 and is a component of approved vaccines. Formulations of poly (I:C) have been described in the literature. For example AU2014345667A1 describes a formulation for administration to the upper respiratory tract. Malcolm et al. (Antiviral Res. 2018 May; 153:70-77) describe a nasal powder comprising poly(I:C) - PrEP-001 - and dosages and modes of administration thereof, which can be used in the present invention.

Another preferred tlr agonist is R848 (resiquimod). Resiquimod is a dual tlr7/8 agonist. Formulations of resiquimod have been described in the literature. For example WO2017/004421A1 describes topical and injectable formulations (the injectable formulations could be useful for systemic administration of resiquimod) and US2004/136917A1 describes formulations for administration to the upper respiratory tract.

Imiquimod is an FDA-approved tlr7 agonist. Formulations of imiquimod have been described in the literature. US2004/136917A1 describes, for example, formulations for administration to the upper respiratory tract.

VTX-2337 (motolimod) is a tlr8 agonist and has been used in several clinical phase II studies for the treatment of various cancer types. In the "Active8" clinical trial a subcutaneous formulation of motolimod was used, but other formulations for systemic administration or administration to the upper respiratory tract are described, for example WO2017/079283A1 describes dosages and formulation.

GLA-SE (glucopyranosyl lipid-A (GLA) in a stable, oil-in-water emulsion) is a tlr4 agonist and has been used as an adjuvant in numerous vaccines. Formulations comprising GLA-SE have been described in the literature. For example WO2015/112485A1 describes formulations and dosages of GLA-SE in combination with an antigen. The skilled person will appreciate that GLA-SE formulations suitable in the methods of the present invention can simply be made by taking a described vaccine formulation comprising GLA-SE and omitting the respective vaccine antigen. In an urgency situation, however, the existing vaccines might even be useful.

SD-101 is a tlr9 agonist - a synthetic CpG oligonucleotide, and has been used in clinical trials (for example see Clinical Trials.gov identifier NCT02521870, where it has been administered by injection).

GSK2245035 (for structural information see Biggadike et al. in J. Med. Chem. 2016, 59, 5, 1711-1726) is a tlr7 agonist and has been used in clinical trials as a nasal spray solution (see, for example ClinicalTrials.gov Identifier: NCT02833974).

RO7119929 is a tlr7 agonist being tested by Roche in a clinical trial against liver cancer, where it is given orally (see, for example, ClinicalTrials.gov Identifier: NCT04338685). WO2015162075A1 describes further tlr7 agonists by Roche.

GSK1795091 is a tlr4 agonist and has been tested in clinical dose-finding trials, where it was administered systemically by intravenous injection (see, for example ClinicalTrials.gov Identifier: NCT02798978).

The present invention also relates to the use of a toll-like receptor agonist in any one of the methods of providing immunity against a virus to a selected subpopulation of a larger population. The tlr receptors, mode of administration, viruses and other variations of the methods can be as described above, in particular as described above for the preferred embodiments.

The present invention also relates to the use of a toll-like receptor agonist for the immunization of a subpopulation of a population during a virus outbreak. The virus outbreak can be a virus epidemic or a pandemic, as in the current example of the SARS-CoV-2 pandemic.

The tlr receptors, mode of administration, viruses and other variations of the methods can be as described above, in particular as described above for the preferred embodiments.

In particular, the present invention relates to the use of a toll-like receptor agonist for the immunization of a subpopulation of a population during a virus outbreak, wherein the toll-like receptor agonist is to be administered to a subpopulation who is at risk of being exposed to the virus within seven days after administration of the toll-like receptor agonist.

In a further embodiment, the present invention relates to a tlr agonist for use in the one-time prophylactic treatment against an infection, such as a virus infection, of a subpopulation, wherein the subpopulation is a collection of asymptomatic subjects in proximity to subjects of the population who show symptoms of infection, in particular virus infection.

Tlr agonists have been described for the prophylactic treatment of virus infections (see, for example, Zhao et al., J. Virol. 2012 Nov, 86(21):11416-11424. Also the tlr agonist Bacillus Calmette-Guerin vaccine has been discussed for Covid-19 prevention.

However, the authors who have suggested tlr agonists for prophylactic treatment fail to appreciate that i) the stimulation of the innate immune system by administration of the tlr agonist only lasts for a short period of time, and/or ii) repeated administration of the tlr agonist leads to a phenomenon called "tlr tolerance", where the body decreases the strength of the antiviral response in reaction to repeated administration of a tlr agonist further and further until the organism might become even more vulnerable to an infection after repeated doses of tlr agonist administration.

In other words, and explained for the example of SARS-CoV-2, administration of the BCG "vaccine" would only have a beneficial effect in the first week after its administration, while after week 1 a protective effect would not be seen, and also not the lasting effect of a "vaccination".

A "one time administration" is administration of the tlr agonist on only one or two subsequent days. This should lead to a single peak of the tlr agonist plasma concentration followed by a complete absence of the tlr agonist in the plasma and at least two more weeks thereafter, where the tlr agonist is not administered. This is in contrast to dosage regimens where administration of the tlr agonist is repeated to keep the plasma level of the tlr agonist at a level above the detection limit.

A preferred example of a one-time administration is where the tlr agonist is administered only within a period of 24 hours, such as within a period of 12 hours or more preferably within a period of 6 hours, 3 hours, 2 hours, 1 hour or 30 minutes. It is most convenient to only administer a single dose of the tlr agonist, such as the tlr agonist in a single tablet, a single injection or a single nasal spray inhalation.

Preferred tlr agonists are a tlr3 agonist, a tlr4 agonist, a tlr7 agonist, a tlr8 agonist and/or a tlr9 agonist. The invention also relates to a tlr agonist for the one-time administration of the present invention, wherein the tlr agonist is selected from the following groups: tlr3, tlr4 and tlr9; tlr3, tlr4, tlr7 and tlr8; tlr4, tlr7, tlr8 and tlr9; tlr7, tlr8 and tlr9; tlr3, tlr7 and tlr8; tlr4, tlr7 and tlr9.

Preferred examples of tlr agonists are selected from the group consisting of polyinosinic:polycytidylic acid, resiquimod, imiquimod, motolimod, GLA-SE, SD-101, GSK2245035, RO7119929, and GSK1795091.

The invention also relates to tlr agonists for the one-time administration of the present invention, wherein the tlr agonist is not polyinosinic:polycytidylic acid. The invention also relates to tlr agonists for the one-time administration of the present invention, wherein the tlr agonist is not resiquimod. The invention also relates to tlr agonists for the one-time administration of the present invention, wherein the tlr agonist is not imiquimod. The invention also relates to tlr agonists for the one-time administration of the present invention, wherein the tlr agonist is not motolimod. The invention also relates to tlr agonists for the one-time administration of the present invention, wherein the tlr agonist is not GLA-SE. The invention also relates to tlr agonists for the one-time administration of the present invention, wherein the tlr agonist is not GSK2245035. The invention also relates to tlr agonists for the one-time administration of the present invention, wherein the tlr agonist is not RO7119929. The invention also relates to tlr agonists for the one-time administration of the present invention, wherein the tlr agonist is not GSK1795091.

The invention also relates to tlr agonists for the one-time administration of the present invention, wherein the virus causes a respiratory disease, such as an RNA-virus, such as a coronavirus, and in particular a SARS-like virus like SARS-CoV-2.

Other preferred viruses are a pestivirus, or an asfarvirus.

The one-time prophylactic treatment of the present invention can be seen as an emergency salvage measure of a group at immediate risk of contracting the infectious agent, such as an infectious bacteria or an infective virus, for example a coronavirus like SARS-CoV-2.

In the case where the population is a human population, such a situation is, for example, when medical personnel of a hospital is about to receive subjects who show symptoms of virus infection, in particular when personal protective equipment is limited or not available. The medical personnel would be at an extremely high risk of contracting the virus in such a situation and would benefit from a tlr agonist, which can boost their immune system during the short time, when the first viral exposure can be expected.

Another emergency situation, where the one time prophylactic treatment of the present invention would help, is a situation where a vulnerable subpopulation, a subpopulation having an infection fatality rate that is known to be or expected to be above the average infection fatality rate of the population, is at immediate risk of contracting the virus. Taking the Covid-19 pandemic as an example, this is the case, when a first person with possible symptoms of Covid-19 is detected in an old people's home. In such a situation, the tlr agonist should be administered to the subpopulation of asymptomatic individuals. In such a situation it must be of concern that these vulnerable individuals might get exposed to the virus in the next minutes of hours, and in such a situation boosting the innate immune system of still asymptomatic individuals is expected to reduce the number of patients who develop severe Covid-19 and who die.

In the case where the population of vertebrates, such as a mammalian animal population, and for example farm animals like pigs, cows, sheep, goats or horses, such an emergency situation can also arise. This will be explained by following example of swine fever, which is a highly lethal viral disease of pigs caused by a flavivirus (in the case of classical swine fever) or an asfarvirus (in the case of African swine fever). It is current practice to cull the complete swine population of a farm where one animal with swine fever has been detected, as the disease is highly contagious among swine and as there is no treatment for the disease. The tlr agonists for the one-time prophylactic treatment of the present invention present invention would allow a different approach when a first pig with possible symptoms of swine fever is detected in a farm. In such a situation, the tlr agonist should be administered to the subpopulation of asymptomatic pigs. In such a situation it must be of concern that these vulnerable pigs would get exposed to the virus in the next minutes of hours, and in such a situation boosting the innate immune system of still asymptomatic pigs is expected to reduce the number of pigs who die from swine fever. This would have the advantage that a part of the swine population would survive the swine fever and recover from it, resulting to a lesser economic loss to the farmer than a culling of the whole population of swine on his farm.

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. As used in the specification and claims, the singular form "a", "an" and "the" include plural references unless the context clearly dictates otherwise.

As used herein "immunization" is the process whereby individuals from a subpopulation are made immune or resistant to a disease caused by a pathogen, such as a virus or a bacterium. If, at least one month after the process of immunization, a group of immunized individuals is challenged with the pathogen against it was immunized, the group of immunized individuals will have lower mortality and/or lower morbidity than a comparable control group of unimmunized individuals challenged with the pathogen under comparable experimental conditions. The "immunized" individuals have gained "immunity", a lower risk of mortality and/or a lower risk of morbidity compared to comparable individuals who are not immune.

As used herein an "agonist" is a chemical that binds to a receptor and activates the receptor to produce a biological response.

As used herein "epidemic" and "pandemic" adhere to the nomenclature of the Center for Disease Control, USA.

As used herein the term "prophylactic treatment" refers to the prevention of the onset, recurrence or spread of a disease or disorder, or of one or more symptoms thereof.

As used herein the term "infection fatality rate" is ratio between events of death among infected people and the total number of infected people.

As used herein "respiratory disease" is a disease of the respiratory tract, typically causing symptoms like difficult breathing, cough, breathing noisily, lingering chest pain and/or coughing up blood.

As used herein "tlr3" is the protein encoded by the human tlr3-gene. The tlr3 gene has the reference sequence ID NG_007278.

As used herein "tlr4" is the protein encoded by the human tlr4-gene. The tlr4 gene has the reference sequence ID NG_011475.

As used herein "tlr7" is the protein encoded by the human tlr7-gene. The tlr7 gene has the reference sequence ID NG_012569.

As used herein "tlr8" is the protein encoded by the human tlr8-gene. The tlr8 gene has the reference sequence ID NG_012882.

As used herein "tlr9" is the protein encoded by the human tlr9-gene. The tlr9 gene has the reference sequence ID NG_033933.

As used herein, the term "proximity" relates to nearness in space, such as being in the same house, on the same farm or in the same hospital.

As used herein, the term "dose" is a quantity of a drug to be taken at a particular time.

As used herein the term "symptomatic" relates to exhibiting characteristics of a particular disease.

As used herein the term "nonsymptomatic" or "asymptomatic" relates to not or not yet exhibiting the characteristics of a particular disease.

As used herein, "at least one" means "one or more."

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human.

The terms "subject" or "patient" do not denote a particular age or sex. Thus, adult, infant and newborn subjects, whether male or female, are intended to be covered.

The term "SARS-CoV-2" is used herein to define an RNA viral species

The term "emergency situation" as used herein is the immediate risk to contract the infectious agent causing the infection.

The invention is further exemplified by the following embodiments
1. A method of providing immunity against a virus to a selected subpopulation of a larger population by
   a) administering an agonist of a toll-like-receptor to individuals from the selected subpopulation, and then
   b) exposing the individuals from step a) to the virus.
2. The method of embodiment 1, where step b) is at most seven days after step a).
3. The method of embodiment 2, where step b) is done from 3 days to immediately after step a).
4. The method of any one of embodiments 1 to 3, wherein the population is a human population.
5. The method of any one of embodiments 1 to 3, wherein the population is a population of mammalian animals.
6. The method of embodiments 4 or 5, wherein the subpopulation has an infection fatality rate that is known to be or expected to be below the average infection fatality rate of the population.
7. The method of embodiment 4 or 5, wherein the subpopulation is at an increased risk of exposure to the virus.
8. The method of embodiment 7 in combination with embodiment 4, wherein the subpopulation is medical personnel.
9. The method of any one of embodiments 1 to 8, wherein the virus causes a respiratory disease.
10. The method of any one of embodiments 1 to 9, wherein the virus is an RNA-virus.
11. The method of embodiment 10, wherein the RNA-virus is a coronavirus.
12. The method of embodiment 11, wherein the coronavirus is a SARS-like virus.
13. The method of embodiment 12, wherein the SARS-like virus is SARS-CoV-2.
14. The method of any one of embodiments 1-13, wherein exposure to the virus in step b) and administration of the toll-like-receptor agonist in step a) are effected to the same part of the body.
15. The method of embodiment 14, wherein the part of the body is the upper respiratory tract.
16. The method of embodiment 15, wherein the part of the body is the nasal cavity.
17. The method of embodiment 5, wherein the virus causes a respiratory disease.
18. The method of embodiment 17, wherein the virus is an RNA-virus.
19. The method according to any one of embodiments 1 to 18, wherein the toll-like receptor is signalling via MyD88 or a mammalian homologue thereof.
20. The method of any one of embodiments 1 to 18, wherein the population is a human population and therein the toll-like receptor is tlr3, tlr4, tlr7, tlr8 and/or tlr9.
21. The method of any one of embodiments 1 to 20, wherein the tlr agonist is a tlr3 agonist, a tlr4 agonist, a tlr7 agonist, a tlr8 agonist and/or a tlr9 agonist.
22. The method of any one of embodiments 1 to 21, wherein the tlr agonist is Poly (I:C), R848, imiquimod, VTX-2337, GLA-SE, SD-101, GSK2245035, RO7119929 or GSK1795091, in particular wherein the tlr-agonist is poly(I:C) and wherein from 1mg to 20mg poly(I:C) are to be administered nasally.
23. Use of a toll-like receptor agonist in the immunization of a subpopulation of a population during a virus outbreak.
24. The use according to embodiment 23, wherein the virus outbreak is a virus epidemic.
25. The use according to embodiments 23 and/or 24, wherein the virus outbreak is a pandemic.
26. The use according to any one of embodiments 23 to 25, wherein the toll-like receptor agonist is to be administered to a subpopulation who is at risk of being exposed to the virus within seven days after administration of the toll-like receptor agonist.
27. The use according to any one of embodiments 23 to 26, wherein the tlr agonist is as defined in any one of embodiments 21 or 22.
28. The use according to any one of embodiments 23 to 27, wherein the virus is as defined in any one of embodiments 9 to 13.
29. The use according to any one of embodiments 23 to 28, wherein the population is a human population.
30. The use according to embodiment 29, wherein the subpopulation has an infection fatality rate that is known to be or expected to be below the average infection fatality rate of the population.
31. The use according to embodiment 30, wherein the subpopulation is at an increased risk of exposure to the virus.
32. The use according to embodiment 31, wherein the subpopulation is medical personnel.
33. The use according to any one of embodiments 23 to 28, wherein the population is a mammalian animal population.
34. The use according to embodiment 33, wherein the subpopulation is at an increased risk of exposure to the virus.
35. The use according to embodiment 34, wherein the subpopulation at an increased risk of exposure to the virus is a collection of asymptomatic animals in proximity to animals who show symptoms of virus infection.
36. A single dose of a tlr agonist for the prophylactic treatment of a virus infection in a subpopulation, wherein the subpopulation is a collection of asymptomatic subjects in proximity to subjects of the population who show symptoms of virus infection.
37. The single dose according to embodiment 36, wherein the population is a human population.
38. The single dose according to embodiments 35 and 36, wherein the subpopulation is medical personnel of a hospital receiving subjects who show symptoms of virus infection.
39. The single dose according to embodiments 35 and 36, wherein the subpopulation has an infection fatality rate that is known to be or expected to be above the average infection fatality rate of the population and wherein.
40. The single dose according to embodiment 36, wherein the population is a mammalian animal population.
41. The single dose according to embodiment 40, wherein the mammalian animals are farm animals.
42. The single dose according to embodiment 41, wherein the farm animals are pigs, cows, sheep, goats or horses.
43. The single dose according to embodiment 42 wherein the farm animals are pigs and wherein the virus is a flavivirus or a asfarvirus.
44. A tlr agonist for use in the one-time prophylactic treatment of a subpopulation against an infection, wherein the subpopulation is a collection of asymptomatic subjects in proximity to subjects of the population who show symptoms of infection.
45. The tlr agonist for use in the one-time prophylactic treatment of embodiment 44, wherein the infection is a virus infection.
46. The tlr agonist for use in the one-time prophylactic treatment according to embodiments 44 or 45, wherein the population is a human population.
47. The tlr agonist for use in the one-time prophylactic treatment according to any one of embodiments 44 to 46, wherein the subpopulation is medical personnel of a hospital about to receive patients who show symptoms of virus infection.
48. The tlr agonist for use in the one-time prophylactic treatment according to any one of embodiments 44 to 46, wherein the subpopulation has an infection fatality rate that is known to be or expected to be above the average infection fatality rate of the population and wherein.
49. The tlr agonist for use in the one-time prophylactic treatment according to embodiments 44 or 45, wherein the population is a mammalian animal population except rodents.
50. The tlr agonist for use in the one-time prophylactic treatment according to embodiment 49, wherein the mammalian animals are farm animals.
51. The tlr agonist for use in the one-time prophylactic treatment according to embodiment 50, wherein the farm animals are pigs, cows, sheep, goats or horses.
52. The tlr agonist for use in the one-time prophylactic treatment according to embodiment 51 wherein the farm animals are pigs and wherein the virus is a flavivirus or an asfarvirus.
53. The tlr agonist for use in the one-time prophylactic treatment according to any one of embodiments 44 to 52 wherein the tlr agonist is a tlr3 agonist, a tlr4 agonist, a tlr7 agonist, a tlr8 agonist and/or a tlr9 agonist.
54. The tlr agonist for use in the one-time prophylactic treatment according to any one of embodiments 44 to 53 wherein the tlr agonist is a tlr3 agonist, in particular wherein the tlr-agonist is poly(I:C) and wherein from 1mg to 20mg poly(I:C) are to be administered nasally.
55. The tlr agonist for use in the one-time prophylactic treatment according to any one of embodiments 44 to 53 wherein the tlr agonist is a tlr4 agonist.
56. The tlr agonist for use in the one-time prophylactic treatment according to any one of embodiments 44 to 53 wherein the tlr agonist is a tlr7 agonist.
57. The tlr agonist for use in the one-time prophylactic treatment according to any one of embodiments 44 to 53 wherein the tlr agonist is a tlr8 agonist.
58. The tlr agonist for use in the one-time prophylactic treatment according to any one of embodiments 44 to 53 wherein the tlr agonist is a tlr9 agonist.
59. The tlr agonist for use in the one-time prophylactic treatment according to any one of embodiments 44 to 53 wherein the tlr agonist is Poly (I:C), resiquimod, imiquimod, VTX-2337, GLA-SE, SD-101, GSK2245035, RO7119929 or GSK1795091, in particular wherein the tlr-agonist is poly(I:C) and wherein from 1mg to 20mg poly(I:C) are to be administered nasally.
60. A tlr agonist for use in the prophylactic treatment of asymptomatic subjects against an infection in an emergency situation.
61. The tlr agonist for use according to embodiment 60, wherein the infection is a virus infection.
62. The tlr agonist for use according to embodiments 60 or 61, wherein the asymptomatic subject is a human being.
63. The tlr agonist for use according to any one of embodiments 60 to 62, wherein the human being is a member of medical personnel of a hospital and wherein the emergency situation is receiving a patient into the hospital who shows symptoms of virus infection.
64. The tlr agonist for use according to any one of embodiments 60 to 63, wherein the asymptomatic subject belongs to a subpopulation having an infection fatality rate that is known to be or expected to be above the average infection fatality rate of the population.
65. The tlr agonist for use according to embodiments 60 or 61, wherein the subject is a mammalian animal, preferably wherein the subject is not a rodent.
66. The tlr agonist for use according to embodiment 65, wherein the mammalian animal is a farm animal.
67. The tlr agonist for use according to embodiment 66, wherein the subject is a pig, a cow, a sheep, a goat or a horse.
68. The tlr agonist for use according to embodiment 67 wherein the subject is a pig and wherein the virus is a flavivirus or an asfarvirus.
69. The tlr agonist for use according to any one of embodiments 60 to 68 wherein the tlr agonist is a tlr3 agonist, a tlr4 agonist, a tlr7 agonist, a tlr8 agonist and/or a tlr9 agonist.
70. The tlr agonist for use according to any one of embodiments 60 to 69 wherein the tlr agonist is a tlr3 agonist, in particular wherein the tlr-agonist is poly(I:C) and wherein from 1mg to 20mg poly(I:C) are to be administered nasally.
71. The tlr agonist for use according to any one of embodiments 60 to 69 wherein the tlr agonist is a tlr4 agonist.
72. The tlr agonist for use according to any one of embodiments 60 to 69 wherein the tlr agonist is a tlr7 agonist.
73. The tlr agonist for use according to any one of embodiments 60 to 69 wherein the tlr agonist is a tlr8 agonist.
74. The tlr agonist for use according to any one of embodiments 60 to 69 wherein the tlr agonist is a tlr9 agonist.
75. The tlr agonist for use according to any one of embodiments 60 to 69 wherein the tlr agonist is Poly (I:C), resiquimod, imiquimod, VTX-2337, GLA-SE, SD-101, GSK2245035, RO7119929 or GSK1795091, in particular wherein the tlr-agonist is poly(I:C) and wherein from 1mg to 20mg poly(I:C) are to be administered nasally.
76. The tlr agonist for use according to any one of embodiments 60 to 75, wherein the emergency situation is the immediate risk to contract the infectious agent causative for the infection.
77. The tlr agonist for use according to any one of embodiments 60 to 75, wherein the emergency situation is the proximity of subjects who show symptoms of infection.
78. The tlr agonist for use according to any one of embodiments 60 to 75, wherein the emergency situation is a newly detected outbreak of the infection in a building where more than one subject lives or works and wherein the subjects are human being.
79. The tlr agonist for use according to any one of embodiments 60 to 75, wherein the emergency situation is a newly detected outbreak of the infection in a building, where subjects live or work who are expected to have an infection fatality rate that is known to be or expected to be above the average infection fatality rate of the population.
80. The tlr agonist for use according to any one of embodiments 60 to 75, wherein the emergency situation is a newly detected outbreak of a virus infection in an old people's care facility and wherein the subjects are human being.
81. The tlr agonist for use according to any one of embodiments 60 to 75, wherein the emergency situation is a newly detected outbreak of a virus infection on a farm and wherein the subjects are farm animals.
82. The tlr agonist for use according to any one of embodiments 60 to 75, wherein the emergency situation is a newly detected outbreak of a swine virus infection on a farm and wherein the subjects are pigs.
83. The tlr agonist for use according to any one of embodiments 60 to 75, wherein the emergency situation is a newly detected outbreak of a swine fever virus infection on a farm, wherein the subjects are pigs and wherein the virus is a flavivirus or an asfarvirus.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### EXAMPLES

### Example 1

The experiment shown in figure 1 A and C of Zhao et al., J. Virol. 2012 Nov, 86(21):11416-11424 is repeated. B6 mice are lightly anesthetized with isoflurane and infected intranasally with 1x10⁵ PFU of SARS-CoV. Aged mice are infected with 10⁵PFU SARS-CoV-1. Mice are first treated with agonists for TLR3 (poly(I·C), TLR4 (LPS), TLR7/8 (resiquimod), and TLR9 (CpG) and then infected with SARS-CoV-1 shortly after pretreatment. Treatment with agonists for TRL3, TLR7/8, and TLR9 all increase survival, pretreatment with poly(I·C) is most protective.

After 10 to 14 days mice show complete virus clearance. After 14 more days, the surviving mice are investigated for the presence of neutralizing antibodys for SARS-CoV-1. Several mice have developed neutralizing antibodies against the SARS-CoV-1 virus.

### Example 2

21 days after complete virus clearance 10 mice which have survived the first challenge with SARS-CoV-1 are again infected with 10⁵PFU SARS-CoV-1. More than one mouse survives this second challenge, indicating that mice which survive the first SARS-CoV-1 challenge with the help of the pretreatment with a tlr agonist develop some immunity against the virus.

### Example 3

In a farm where a first case of classical swine fever is reported among a population of at least 100 pigs, all but one symptomatic pigs are culled. 30 apparently unsymptomatic pigs are treated intranasally with 0.1 mg/kg poly (I:C) and marked so that these pretreated individuals can be identified. One hour after the pretreatment the whole population of apparently unsymptomatic pigs, which includes at least 30 unsymptomatic pigs which have not received a pretreatment, is allowed to mingle with the infected pig for 24 hours. The infected pig is then culled and removed and the pretreated pigs are then separated from the rest of the population. Pigs who develop symptoms of severe classical swine fever are culled and counted as dead. The infection fatality rate is determined for the subpopulation of poly (I:C)-pretreated pigs and for the subpopulation which did not receive a pretreatment. The infection fatality rate for the subpopulation of pretreated pigs is lower than the infection fatality rate of the untreated subpopulation.

### Example 4

In an old people's home where the first patient with COVID-19 symptoms is detected, to half of the still asymptomatic inhabitants (number of all inhabitants is at least 20) of age 65 or higher 6.4 mg (4x800µg per nostril using a dry powder applicator) of the tlr3 agonist PrEP-001 (contains poly(I:C)) are administered once, similar to what has been described in detail in the context of a clinical trial in Malcom et al., Antiviral Res. 2018 May; 153:70-77. The other half of the still asymptomatic patients receive placebo dry powder. After three weeks, there are fewer deaths and/or fewer severe cases requiring intensive care in the treatment group vs. the placebo group.

## Claims

1. A tlr agonist for use in the one-time prophylactic treatment of a subpopulation against an infection, wherein the subpopulation is a collection of asymptomatic subjects in proximity to subjects of the population who show symptoms of infection.

2. A method of providing immunity against a virus to a selected subpopulation of a larger population by
a) administering an agonist of a toll-like-receptor to individuals from the selected subpopulation, and then
b) exposing the individuals from step a) to the virus.

3. The method of claim 2, where step b) is at most seven days after step a).

4. The method of any one of claims 2 to 3, wherein the population is a human population.

5. The method of any one of claims 2 to 4, wherein the subpopulation has an infection fatality rate that is known to be or expected to be below the average infection fatality rate of the population.

6. The method of claim 5, wherein the subpopulation is **characterized by** an age of at most 40 years.

7. The method of any one of claims 2 to 6, wherein the virus is a coronavirus.

8. The method of claim 7, wherein the coronavirus is SARS-CoV-2.

9. The method of any one of claims 2-8, wherein exposure to the virus in step b) and administration of the toll-like-receptor agonist in step a) are effected to the same part of the body.

10. The method of claim 9, wherein the part of the body is the nasal cavity.

11. The method of any one of claims 2 to 10, wherein the population is a human population and wherein the toll-like receptor is tlr3, tlr4, tlr7, tlr8 and/or tlr9.

12. The method of any one of claims 2 to 11, wherein the tlr agonist is a tlr3 agonist, a tlr4 agonist, a tlr7 agonist, a tlr8 agonist and/or a tlr9 agonist.

13. The method of any one of claims 11 to 12, wherein the tlr agonist is Poly (I:C), R848, imiquimod, VTX-2337, GLA-SE, SD-101, GSK2245035, RO7119929 or GSK1795091.

14. Use of a toll-like receptor agonist in the immunization of a subpopulation of a population during a virus outbreak.
